(19) Europäisches Patentamt

European Patent Office

Office européen des brevets

(11) Publication number: **0 040 938**
A2

## (12) EUROPEAN PATENT APPLICATION

(21) Application number: **81302170.6**

(22) Date of filing: **15.05.81**

(51) Int. Cl.³: **A 61 K 7/16**

(30) Priority: **19.05.80 US 150870**

(43) Date of publication of application: **02.12.81**
Bulletin 81/48

(84) Designated Contracting States: **BE DE FR GB NL SE**

(71) Applicant: **MONSANTO COMPANY, Patent Department 800 North Lindbergh Boulevard, St. Louis, Missouri 63166 (US)**

(72) Inventor: **Jarvis, William Martin, 822 Clark Avenue, Webster Groves Missouri 63119 (US)**
Inventor: **Schiff, Thomas, 8149 Pershing Avenue, Clayton Missouri 63105 (US)**

(74) Representative: **Lunt, John Cooper et al, Monsanto House 10-18 Victoria Street, London, SW1H ONQ (GB)**

(54) **Prophylactic dental paste comprising dicalcium phosphate and method of using it for cleaning, polishing and remineralizing teeth.**

(57) A prophylactic dental paste for professional use containing at least 60 percent by weight of dicalcium phosphate, (which may be anhydrous dicalcium phosphate, dicalcium phosphate dihydrate or a mixture of the two) not only satisfactorily cleans the tooth surface but provides a tooth with higher luster or polish. The use of the prophylatic dental paste of the invention, in which the dicalcium phosphate has the same calcium to phosphorus atom ratio found in the tooth, aids in remineralization of the cleaned tooth surface.

PROPHYLACTIC DENTAL PASTE COMPRISING DICALCIUM
PHOSPHATE AND METHOD OF USING IT FOR CLEANING,
POLISHING AND REMINERALIZING TEETH.

This invention relates to a new composition for cleaning and polishing teeth by dental prophylaxis and more specifically to a dental prophylactic paste comprising dicalcium phosphate dihydrate.

Dental plaque, food particles, exogenous stains and other tooth surface pigmentation can be removed, to varying degrees, from teeth by means of a suitable dentifrice and toothbrush during ordinary daily brushings. Some enamel stains and pigmentation, however, are too resistant to the abrasives found in conventional dentifrice formulations. As a result, dentists must perform a "prophylaxis" in order to remove not only dental calculus (tartar) but the accumulated stains not satisfactorily removed by the daily use of a dentifrice. Most frequently, when either a dentifrice or a common cleansing and polishing agent used to perform a prophylaxis are employed, the teeth may be cleaned but they exhibit a low luster or polish. For good dental aesthetics, clean teeth with a high luster are desired and it would be desirable to provide a cleaning and polishing agent capable of accomplishing these results.

The problems encountered in the relatively infrequent use of a cleaning and polishing agent in a prophylactic paste used for a prophylaxis performed once or twice a year are much different than the problems encountered in the use of a cleaning and polishing agent as a constituent of a dentifrice for use on a daily basis, even though the desired end result of cleaning and polishing of tooth enamel may be the same. Obviously, it is possible to use a more highly abrasive agent in a prophylactic paste than in a daily brushing with a conventional dentifrice. However, care must be taken in both cases to avoid extensive enamel loss at the expense of cleaning the teeth. In general, it has been assumed that in attempting to remove the more difficult forms of stain, such as tobacco, tea, coffee and similar stains, there must be a major compromise between cleaning and tooth structure loss, i.e., that in order to have maximum removal of these more difficult stains and/or pigmentations, the tooth structure must be sacrificed. Thus, it would be desirable to provide a cleaning and polishing composition for dental prophylaxis which effectively removes even the most difficult enamel stains and pigmentation while minimizing enamel loss due to abrasion.

Another factor to be considered in a suitable prophylactic composition is the ability to polish the teeth to a high luster, i.e., to achieve a smooth and highly lustrous enamel surface. Highly polished surfaces apparently are less receptive to retention of plaque and oral debris, and this factor is one of the motivating reasons for developing a prophylactic paste which not only cleans effectively but which produces an exceptionally good luster.

All of the commonly used prophylactic dental paste compositions in the prior art either suffer from the disadvantage of inadequate cleaning or inadequate polishing of the dental surface. For example, flour of pumice is commonly used in dental prophylactic paste compositions and it is a fair cleanser but it is highly abrasive and produces an extremely poor polish. In fact, the resulting tooth surface is so rough after being treated with flour of pumice that the occurrence of dental calculus, stains, pellicle and other oral debris is significantly increased. Alumina is an excellent polishing agent which produces a very smooth and shiny surface, but it is so abrasive that the resultant tooth structure loss precludes its recommended use. Precipitated chalk and zirconium silicate, on the other hand, clean fairly well but produce a rough tooth surface. A mixture of insoluble sodium metaphosphate and tricalcium phosphate is a fairly effective polishing agent but the combination is not a particularly good cleaning agent. It has been disclosed in U.S. 3,378,445 and U.S. 3,330,732 that dicalcium phosphate dihydrate fails to effectively polish.

Thus, it would be highly desirable to provide an effective cleaning and polishing agent which minimizes damage to the tooth structure. As indicated above, a variety of compounds are known which clean well but which do not polish, or which polish well but do not clean satisfactorily. Now, according to the present invention, there is provided a prophylactic dental paste composition comprising dicalcium phosphate which effectively cleans teeth and polishes the enamel surface to a high luster. In addition, dicalcium phosphate dihydrate has been reported to be an effective remineralization agent for enamel surfaces because of the calcium to phosphate mole ratio, and it is believed that the cleaned tooth surface in contact with concentrated dicalcium phosphate effectively aids in the remineralization of the tooth surface. These advantages and results are particularly surprising in light of the prior art which discloses that dicalcium phosphate is a poor polishing agent.

SUMMARY OF THE INVENTION

These and other advantages are achieved by a prophylactic dental paste comprising greater than about 70 percent by weight of dicalcium phosphate, based on the total weight of the paste. There is also provided a prophylaxis method comprising applying to a tooth surface a dental prophylactic paste containing at least about 60 percent by weight dicalcium phosphate, based on the total weight of the paste.

The term "dicalcium phosphate" as it is used in the specification and claims shall mean dicalcium phosphate dihydrate, anhydrous dicalcium phosphate and mixtures thereof.

Dicalcium phosphate, and particularly dicalcium phosphate dihydrate, is commonly used as a polishing agent in dentifrices to clean teeth with a toothbrush. Broadly described, dicalcium phosphate dihydrate is prepared by the addition of a lime slurry to phosphoric acid under conditions such that dicalcium phosphate dihydrate is precipitated. In many cases, the dicalcium phosphate also contains other ingredients to inhibit spontaneous hydrolysis and/or dehydration. Stabilizing agents such as alkali metal pyrophosphate and trimagnesium phosphate are added to the dicalcium phosphate dihydrate during its preparation to stabilize the dicalcium phosphate dihydrate. Processes for preparing dicalcium phosphate stabilized against spontaneous hydrolysis and/or dehydration have been disclosed in U.S. 2,287,699, U.S. 3,012,852 and U.S. 4,193,973.

As is known to those skilled in the art, anhydrous dicalcium phosphate can be prepared in accordance with the teachings in U.S. 3,647,371 by adding lime to aqueous phosphoric acid while the reaction temperature is maintained sufficiently high to insure production of dicalcium phosphate substantially free from its water of crystallization.

The dicalcium phosphate useful in the prophylactic dental paste of the present invention can be dicalcium phosphate dihydrate or anhydrous dicalcium phosphate or mixtures of the two. However, as is known to those skilled in the art, anhydrous dicalcium phosphate frequently has a radioactive dentine abrasivity (RDA), as determined according to the method of Grabenstetter et al, JOURNAL OF DENTAL RESEARCH, Vol. 37, page 1060 (1958) of from about 1,000 to 1,400. Hence, the use of anhydrous dicalcium phosphate with such high abrasivities should be avoided in the prophylactic dental paste of the present invention to avoid loss of hard tooth structure due to abrasion. The dicalcium phosphate in the prophylactic dental paste should have an RDA of less than 1,000 and it is preferred that the paste have an RDA of less than about 750. As is known to those skilled in the art, anhydrous dicalcium phosphate having an RDA value of about 50 to about 150 has been prepared according to the teachings of U.S. 3,647,371 and such anhydrous dicalcium phosphate is suitable for use in the paste of the present invention. On the other hand, dicalcium phosphate dihydrate has an RDA value of less than 500 and its use is preferred in the paste of the present invention. However, as will occur to those skilled in the art in view of the present disclosure, a mixture of dicalcium phosphate dihydrate and anhydrous dicalcium phosphate can be used, particularly if the anhydrous dicalcium phosphate has an RDA value of less than 750, as the dicalcium phosphate for the paste of the present invention, and a mixture of dicalcium phosphate dihydrate and anhydrous dicalcium phosphate can be used if higher abrasivity is required provided that the RDA value of the prophylactic dental paste is less than 1,000 and preferably less than 750.

The prophylactic dental paste of the present invention should contain at least about 60 percent by weight of dicalcium phosphate, preferably greater than 70 percent by weight dicalcium phosphate, to insure that there is adequate polishing agent for the dental prophylaxis to remove stains and other dental debris. On the other hand, a paste containing more than about 90 percent by weight dicalcium phosphate, based on the total weight of the paste, can become so viscous that it is difficult to apply to the teeth using conventional cleaning techniques. Thus, it is preferred that the paste contain between about 75 weight percent and 85 weight percent, based on the total weight of the paste, to provide a paste having sufficient polishing agent to effectively clean the teeth while maintaining a sufficiently low viscosity to permit ease of use.

The balance of the formulation is water and optionally other ingredients that are typically used in a prophylactic dental paste, such as flavoring agents like oil of wintergreen, oil of peppermint, oil of spearmint, oil of sassafras, oil of anise and the like; sweetening agents such as saccharin, dextrose, levulose, sodium cyclamate and the like; binders such as water soluble salts of cellulose ethers such as sodium carboxymethyl cellulose and sodium carboxymethyl hydroxyethyl cellulose; natural gums such as gum arabic and the like; humectants such as glycols, glycerin, sorbitol and other polyhydric alcohols; and coloring agents. The prophylactic dental paste of the present invention can be used in a conventional manner to clean teeth professionally using conventional equipment and techniques. The optimum amount of paste, the degree of cleaning and polishing desired, the RDA of the paste and the like can be determined in accordance with conventional techniques.

When teeth are cleaned professionally to remove plaque, tartar, pellicle and other dental debris and the enamel of the teeth is exposed, it is frequently desirable to treat the teeth with fluoride to inhibit caries formation. Accordingly, the paste can optionally contain a source of fluoride ion, such as monofluorophosphate and the like, to aid in this treatment. In addition, although Applicants do not wish to be bound by any particular theory, it is believed that the dicalcium phosphate used in the prophylactic dental paste of the present invention in contact with the teeth free of tartar, plaque and pellicle, aids in the remineralization of the tooth surface, an advantage not found with the prophylactic dental paste compositions of the prior art.

DESCRIPTION OF THE PREFERRED EMBODIMENTS

This invention is illustrated by but not limited to the following Examples wherein all percentages are by weight unless otherwise noted.

EXAMPLE I

A carrier gel is prepared by mixing the following ingredients in the indicated amounts:

| INGREDIENT | WEIGHT (Grams) |
|---|---|
| Glycerin | 1938.4 |
| Carboxymethyl Cellulose | 79.2 |
| Methyl Paracept | 3.6 |
| Propyl Paracept | 0.8 |
| Saccharin | 17.6 |
| Water | 1960.4 |

A series of prophylactic dental pastes are each prepared by combining 75 grams of the above carrier gel with 175 grams of the following polishing agents: (1) chalk; (2) flour of pumice; (3) zirconium silicate; and (4) dicalcium phosphate dihydrate containing 0.6 percent tetrapotassium pyrophosphate and 2 percent trimagnesium phosphate stabilizers, and having an RDA of about 250.

The above pastes are used in a comparative study. The teeth of patients with stain or enamel pigmentation are cleaned with the four pastes. Either the respective left anterior maxillary central incisor, right anterior maxillary central incisor or the two mandibular central incisors are cleaned with the respective abrasives. The patients are randomly divided in order to evaluate the cleaning and polishing abilities of the four abrasives by randomly selecting the different morphological types of teeth for evaluation by the different abrasives. The teeth are polished using conventional techniques using a dental rubber prophylaxis cup and the four pastes.

Analysis of the tooth surfaces using photomicrographs reveals that the teeth are cleaned of plaque, tartar, pellicle and other dental debris in all cases. However, the extent of damage to the enamel using the conventional abrasives is far more severe than when using the paste containing dicalcium phosphate dihydrate of the present invention which provides a clean tooth surface with high luster.

## EXAMPLE II

Nine extracted teeth are cleaned using conventional techniques with the dicalcium phosphate dihydrate prophylactic dental paste of Example I. The hardness of each tooth is measured using a Knoop microhardness tester. Then, the teeth are soaked in a lactic acid solution at 37°C. for four hours to soften the teeth, and the hardness of each tooth is remeasured. Thereafter the teeth are placed in an agitated aqueous slurry containing 0.143 gram of dicalcium phosphate dihydrate per 100 milliliters of water at 37°C. for 20 hours. The teeth are removed from the slurry and the hardness of each tooth is measured again. The results indicate that contact with the dicalcium phosphate dihydrate slurry increases the hardness of the teeth from about 50 percent to about 80 percent of the original hardness of the cleaned teeth.

Thus it can be seen that in one embodiment of this invention there is provided a method for remineralization of teeth which comprises cleaning the teeth with a prophylactic dental paste, and contacting the cleaned teeth with dicalcium phosphate dihydrate.

Although the invention has been described in terms of specified embodiments which are set forth in considerable detail, it should be understood that this is by way of illustration only and that the invention is not necessarily limited thereto since alternative embodiments and operating techniques will become apparent to those skilled in the art in view of the disclosure. Accordingly, modifications are contemplated which can be made without departing from the spirit of the described invention.

0040938

WHAT IS CLAIMED IS:

1. A prophylactic dental paste comprising greater than about 70 percent by weight dicalcium phosphate, based on the total weight of the paste.

2. A paste of Claim 1 wherein the amount of dicalcium phosphate is greater than about 70 percent but less than about 90 percent.

3. A paste of Claim 1 wherein the amount of dicalcium phosphate is from about 75 percent to about 85 percent.

4. A paste of Claim 1 wherein the dicalcium phosphate has a radioactive dentine abrasivity of less than about 1,000.

5. A paste of Claim 1 wherein the dicalcium phosphate has a radioactive dentine abrasivity of less than about 750.

6. A paste of Claim 1 wherein the dicalcium phosphate is a mixture of dicalcium phosphate dihydrate and anhydrous dicalcium phosphate.

7. A paste of Claim 1 wherein the dicalcium phosphate is dicalcium phosphate dihydrate.

8. A prophylaxis method comprising applying to a tooth surface a dental prophylactic paste containing at least about 60 percent by weight dicalcium phosphate, based on the total weight of the paste.

9. A method of Claim 8 wherein the paste contains from about 70 percent to about 85 percent dicalcium phosphate dihydrate.

10. A method for remineralization of teeth which comprises cleaning the teeth with a prophylactic dental paste, and contacting the cleaned teeth with dicalcium phosphate dihydrate.

11. A method of Claim 10 wherein the prophylactic dental paste contains greater than about 60 percent by weight dicalcium phosphate dihydrate, based on the total weight of the paste.